# EUROPÄISCHE PATENTSCHRIFT

(11) **EP 1 766 419 B1**
(45) Veröffentlichungstag und Bekanntmachung des Hinweises auf die Patenterteilung: **16.09.2009**
(21) Anmeldenummer: 05755655.7
(22) Anmeldetag: 04.06.2005
(51) Int. Cl.: G01N 35/10, G01N 35/00, B01L 3/02, C12M 1/26

(54) **VORRICHTUNG ZUR AUTOMATISCHEN ISOLIERUNG UND BEHANDLUNG VON ZELLKLONEN**
DEVICE FOR AUTOMATICALLY ISOLATING AND TREATING CELL CLONES
DISPOSITIF D'ISOLEMENT ET DE TRAITEMENT AUTOMATIQUE DE CLONES DE CELLULES

(30) Priorität: 07.06.2004 DE 102004027662; 25.09.2004 DE 102004046740
(43) Veröffentlichungstag der Anmeldung: 28.03.2007
(73) Patentinhaber: Aviso GmbH Mechatronic Systems, 07747 Jena (DE)
(72) Erfinder: BORNMANN, Gerd, 99427 Weimar (DE)
(74) Vertreter: Wablat, Wolfgang
(86) Internationale Anmeldenummer: PCT/EP2005/006021
(87) Internationale Veröffentlichungsnummer: WO 2005/121807

(56) Entgegenhaltungen:
- EP-A- 0 571 100
- EP-A- 0 801 309
- EP-A- 1 291 658
- WO-A-03/101586
- GB-A- 1 574 643

## Beschreibung

Die Erfindung betrifft eine Vorrichtung zur automatischen
Isolierung und Behandlung von Zellklonen mittels Klonierglocke zur Trennung des jeweiligen Zellklons von der Umgebung, welche einen Werkzeugkopf aufweist, wobei die Klonierglocke von einer Pipettenspitze lösbar gehalten und selbige an einer konischen Aufnahme des Werkzeugkopfes durch Kraftschluss fixiert ist, weiterhin zum Halten der Klonierglocke an der Pipettenspitze diese einen konischen Abschnitt aufweist, über welchen ein komplementärer konischer Abschnitt am oberen Teil der Klonierglocke gestülpt und der Werkzeugkopf an einem Arm eines Robotsystems befestigt ist, gemäß Oberbegriff des Patentanspruchs 1.

Zur Zellernte besteht die Möglichkeit, die jeweilige Zellkultur punktuell zu isolieren. Hierbei wird mit sogenannten Klonierringen, gearbeitet. Klonierringe werden um das zu erntende Objekt gestülpt und es wird mit einer Spritze Lösungsmittel gezielt hinzugegeben.
Nach einer definierten Zeitspanne kann dann die Zellkultur aufgenommen und verbracht werden.
Während der gesamten Prozedur muss gemäß den Lösungen des Standes der Technik der Klonierring von Hand gehalten und auf den Gefäßboden aufgedrückt werden. Bereits geringe Bewegungen im Sinne eines Verrutschens führen zum Misserfolg der Ernte.

Der Bereich der Ernte ist trotz der Verwendung von Klonierringen noch relativ groß, so dass die Dichte der Zellklone auf dem Gefäßboden gering sein muss, um nur die gewünschten Zellklone zu ernten und eine Verunreinigung zwischen den Kulturen zu vermeiden.

Bei automatisierten Vorrichtungen wird mit Hilfe eines Robotsystems ein Werkzeugkopf mit einer Aufnahmeeinrichtung über die Zellkultur positioniert, wobei für die Positionierung vorab die Lage der Kultur mittels optoelektronischer Mittel ermittelt wird.

Der vom Robotsystem gesteuerte Werkzeugkopf führt dann eine Bewegung in Z-Richtung, d.h. im wesentlichen vertikal zur Zellkultur hin aus und nimmt den jeweiligen Klon auf, um diesen dann zur weiteren Untersuchung und Kultivierung an einen anderen Ort zu transportieren.

Die GB-A-1 574 643 offenbart eine Vorrichtung zur automatischem Behandlung von zellen. Die Vorrichtung weist einen Werkzengkopf mit einer konischen Aufnahme auf, an welcher eine Pipettenspitze durch Kraftschluss fixiert werden kann.

Zur Aufnahme und zum Transport des zu erntenden Zellklons kommen Pipettenspitzen aus Kunststoff zum Einsatz, welche mit dem zu verwendenden Lösungsmittel gefüllt werden. Eine Klonierglocke wird über die Pipettenspitze gestülpt. Mit der Klonierglocke kann der zu erntende Zellklon kleinflächig von der Umgebung isoliert und dadurch getrennt werden. Durch die Zugabe des Lösungsmittels aus der Pipettenspitze, einem anschließenden Spülen und Einwirken des Lösungsmittels wird dann der Zellklon vom Gefäßboden gelöst und in die Pipettenspitze gesaugt. Hiernach erfolgt das vorstehend erwähnte Transportieren des Zellklons in ein anderes Gefäß, beispielsweise einer Titerplatte zum Zweck der weiteren Untersuchung.
Die speziellen Pipettenspitzen mit Klonierglocke sind unter der Bezeichnung "Clonetip" (eingetragene Marke) erhältlich.

Es hat sich gezeigt, dass bei dem Ausführen der Bewegung des Werkzeugkopfes zur Aufnahme der Pipettenspitze mit Klonierglocke bestimmte Druckkräfte zum Erreichen eines ausreichenden Kraftschlusses der jeweiligen konischen Verbindungen sicher erreicht werden müssen. Diese Kräfte müssen jedoch in einer solchen Weise exakt eingehalten werden, dass beim Abziehen der Klonierglocke nur diese, nicht jedoch die gesamte Pipettenspitze mit darin enthaltenem, in der Flüssigkeit befindlichen Zellklon entfernt wird.

Aus dem Vorgenannten ist es daher Aufgabe der Erfindung, eine weiterentwickelte Vorrichtung zur automatischen Isolierung und Behandlung von Zellklonen mittels Klonierglocke zur Trennung des jeweiligen Zellklons von der Umgebung anzugeben, welche einen Werkzeugkopf aufweist, wobei die Klonierglocke von einer Pipettenspitze lösbar gehalten und selbige an einer konischen Aufnahme des Werkzeugkopfes durch Kraftschluss fixiert ist. Aufgabengemäß soll der Werkzeugkopf so ausgestaltet werden, dass bei der durch ein Robotsystem durchgeführten Aufnahmebewegung für die Klonierglocke und Pipettenspitze solche Kräfte sich zur Halterung der einzelnen Teile am Werkzeugkopf ergeben, dass bei einem späteren Abziehen der Klonierglocke nur diese, nicht jedoch die eigentliche Pipettenspitze entfernt wird.

Die Lösung der Aufgabe der Erfindung erfolgt mit einem Werkzeugkopf in seiner Merkmalskombination gemäß der Lehre nach Patentanspruch 1, wobei die Unteransprüche mindestens zweckmäßige Ausgestaltungen und Weiterbildungen darstellen.

Demnach ist erfindungsgemäß am werkzeugkopfseitigen Ende des Armes, welcher mit einem Robotsystem in Verbindung steht, ein Aufnahmeblock vorgesehen, welcher einen unteren und einen oberen Anschlag bei einer Bewegung in vertikaler bzw. Z-Richtung als Verschiebewegbegrenzung einer beweglichen Halterung zur Pipettenspitzenaufnahme aufweist.

Die Halterung ist am Aufnahmeblock mittels einer Linearführung verschieblich geführt.

Der obere Anschlag ist als gestaffelte, einseitig eingespannte Blattfedergruppe ausgebildet, so dass sich über den konstant vorgegebenen Verschiebeweg in vertikaler bzw. Z-Richtung bei einer Bewegung des Armes mit Werkzeugkopf gegen einen Fixpunkt verschiedene, gestufte Anschlagkräfte ergeben, welche zu einem definierten Kraftschluss der konischen Halterungen von Pipettenspitze und Klonierglocke führen.

Bei einer Aufnahme des vorbereiteten Clonetips haftet in der gewünschten Weise zum einen der eigentliche Tip an der Pipettenspitze und zum anderen der Aufnahmeteil der Pipettenspitze am komplementären Gegenstück des Werkzeugkopfes. Wird bei einer nachfolgenden Entfernung des Clonetips eine Zugkraft an diesem ausgeübt, so löst sich zunächst nur die konische Verbindung des Clonetips mit dem entsprechenden konischen unteren Teil der Pipettenspitze. Letztere haftet jedoch noch an ihrem diesbezüglichen Halteteil des Werkzeugkopfes.

Die Pipettenspitzenhalterung ist als Hohlzylinder mit Schlauchanschluss ausgeführt, wobei der Hohlzylinder am unteren Ende außenumfangsseitig einen Konus zur kraftschlüssigen Aufnahme der Pipette aufweist.

Im Aufnahmeblock sind Bolzen zur Befestigung der Blattfedern vorgesehen. Diese Blattfedern werden, wie bereits erwähnt, einseitig eingespannt und über die Bolzen unter Zuhilfenahme von Hülsenteilen aufgenommen, wobei die Hülsenteile gleichzeitig Abstandshalter für die Blattfedern darstellen.

Eine erste, untere Blattfeder stützt sich unmittelbar am Aufnahmeblock ab bzw. liegt dort auf und weist zur beweglichen Halterung hin gerichtet eine einfache oder doppelte Kröpfung auf.

Eine zweite Blattfeder ist über ein erstes Hülsenteil gehalten, wobei sich eine dritte Blattfeder oberhalb der zweiten Blattfeder über ein zweites Hülsenteil abstützt.

Die Blattfedern können als jeweils seitlich beabstandete Federpaare ausgebildet sein.

Bei Biegebelastung der ersten, unteren Blattfeder bewegt sich ein Kröpfungsabschnitt dieser nach oben und kommt mit dem darüber liegenden Ende der zweiten Blattfeder in Kontakt. Deren Biegebewegung überträgt sich dann auf die dritte, darüber liegende Blattfeder mit der Folge, dass bei Ausführung der Bewegung sich eine gestufte Anschlagskraft ergibt, und zwar mit zunehmendem Bewegungsweg in steigender Art.

Der Hohlzylinder, welcher der Pipettenspitzenhalterung dient, kann am konischen Ende noch radial verlaufende Ausnehmungen bzw. nutartige Rücksprünge besitzen, so dass sich ein dichter und fester Sitz des betreffenden Endes der Pipettenspitze einstellt. Die konische Paarung von Klonierglocke und unterem Ende der Pipettenspitze ergibt sich in der Werkstoffkombination Kunststoff und Kunststoff, wobei sich die konische Paarung vom oberen Ende der Pipettenspitze hin zum konischen Teil der Pipettenspitzenhalterung zu Kunststoff und Metall einstellt.

Die Erfindung soll nachstehend anhand eines Ausführungsbeispiels sowie unter Zuhilfenahme von Figuren näher erläutert werden.

Hierbei zeigen:
- Fig. 1: eine Dreiseitenansicht des Werkzeugkopfes mit Blattfederanschlag und
- Fig. 2: eine perspektivische Darstellung des Werkzeugkopfes nach Fig. 1.

Der in den Figuren dargestellte Werkzeugkopf ist Bestandteil einer Vorrichtung zur automatischen Isolierung und Behandlung von Zellklonen mittels Klonierglocke zur Trennung des jeweiligen Zellklons von der Umgebung, wobei die nicht dargestellte Klonierglocke an einer nicht dargestellten Pipettenspitze lösbar gehalten und selbige an einer konischen Aufnahme des Werkzeugkopfes durch Kraftschluss fixiert ist.
Zum Halten der Klonierglocke an der Pipettenspitze weist diese einen konischen Abschnitt auf, über welchen ein komplementärer konischer Abschnitt am oberen Teil der Klonierglocke gestülpt ist.

Am werkzeugkopfseitigen Ende des nicht dargestellten Armes des Robotsystems ist ein Aufnahmeblock 1 vorgesehen, welcher einen unteren Anschlag 2 und einen oberen Anschlag 3 aufweist, welcher in Form einer Blattfederanordnung ausgeführt ist.

Der untere und der obere Anschlag 2, 3 dienen als Verschiebewegbegrenzung einer beweglichen Halterung 4 zur Pipettenspitzenaufnahme, wobei die bewegliche Halterung 4 am Aufnahmeblock 1 z.B. mittels einer Linearführung 5 geführt ist.

Durch die Ausbildung des oberen Anschlags als gestaffelte, einseitig eingespannte Blattfedern ergibt sich über einen konstant vorgegebenen Verschiebeweg bei einer Bewegung des Armes mit Werkzeugkopf eine Stufung der Anschlagskräfte. Die Verschiebebewegung der beweglichen Halterung 4 ist mit der Pfeildarstellung symbolisiert.

Die eigentliche Pipettenspitzenhalterung ist als Hohlzylinder 6 mit Schlauchanschluss 7 ausgebildet, wobei der Hohlzylinder am unteren Ende außenumfangsseitig einen Konus 8 aufweist. In diesem konischen Bereich sind zur Verbesserung des Sitzes der Pipettenspitze radial umlaufende Nuten 9 vorgesehen.

Im Aufnahmeblock 1 befinden sich Bolzen 10, welche verschiedene Hülsenteile 11 als Abstandshalter für die Blattfedern 12, 13 und 14 aufnehmen.

Die erste untere Blattfeder 12 liegt unmittelbar am Aufnahmeblock 1 an und weist zur beweglichen Halterung 4 hin gerichtet eine spezielle Kröpfung auf. Diese Kröpfung vollzieht sich zunächst in einem rechten Winkel nach oben. Hiernach wird entgegengesetzt rechtwinklig eine weitere Abwinklung vorgenommen, die dann in ein V-förmiges Ende 15 der Blattfeder 12 übergeht. Die Spitze des V-förmigen Endes zeigt hierbei zur beweglichen Halterung 4 hin.

Die zweite Blattfeder 13 ist über das erste Hülsenteil 11 gehalten und die dritte Blattfeder 14, welche sich oberhalb der zweiten Blattfeder 13 befindet, wird über ein zweites Hülsenteil 11.1 abgestützt.

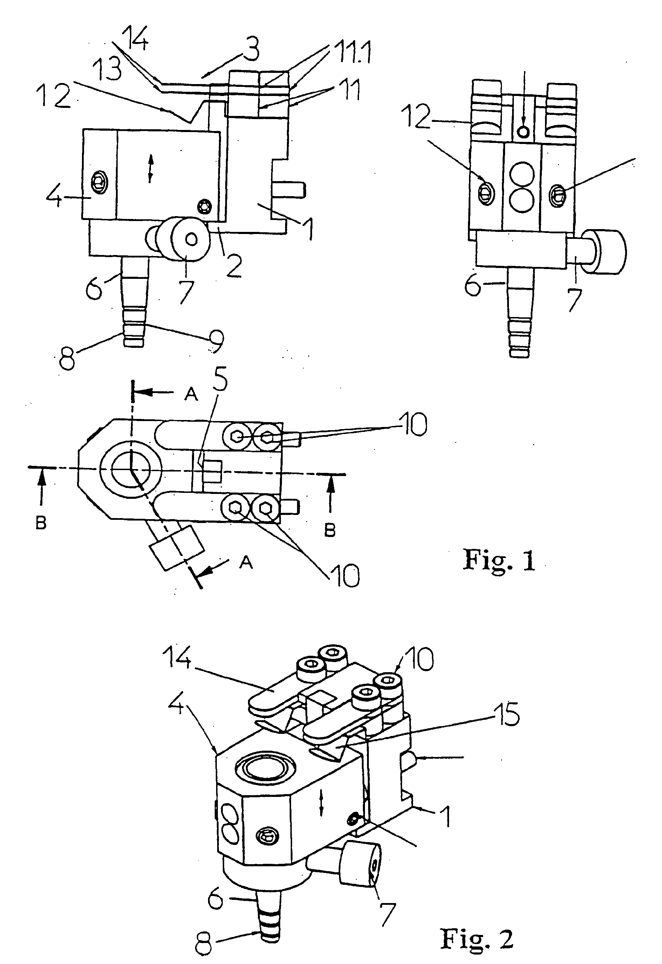

Die Blattfedern können, wie dies insbesondere die perspektivische Ansicht nach Fig. 2 deutlich macht, als seitlich beabstandete Federnpaare ausgebildet werden.

Bei Biegebelastung der ersten, unteren Blattfeder 12 kommt zunächst ein Kröpfungsabschnitt mit dem darüber liegenden freien Ende der zweiten Blattfeder 13 in Kontakt. Deren Biegebewegung überträgt sich dann auf die dritte, darüber liegende Blattfeder 14.

Die Aufnahme der Pipettenspitze mit Clonetip erfolgt dadurch, dass mit Hilfe des nicht dargestellten Robotsystems der Werkzeugkopf über ein entsprechendes Vorratsgefäß geführt wird. Durch Absenken kommt dann der Hohlzylinder 6 mit seinem konischen Ende 8 in Kontakt mit einem komplementären Öffnungsende der Pipettenspitze. Mit weiterer Ausführung der Bewegung in vertikaler Richtung nach unten findet dann der gewünschte Kraftschluss an den konischen Verbindungsabschnitten statt.

Durch die besondere Ausbildung des oberen Anschlags mittels gestaffelter Blattfedern ist dann bei dem Abziehen des Clonetips sichergestellt, dass nur dieser, nicht jedoch die Pipettenspitze mit darin befindlichem Zellklon von der Halterung entfernt wird.

## Patentansprüche

1. Vorrichtung zur automatischen Isolierung und Behandlung von Zellklonen mittels Klonierglocke zur Trennung des jeweiligen Zellklons von der Umgebung, welche einen Werkzeugkopf aufweist, wobei die Klonierglocke von einer Pipettenspitze lösbar gehalten und selbige an einer konischen Aufnahme(8) des Werkzeugkopfes durch Kraftschluss fixiert ist, weiterhin zum Halten der Klonierglocke an der Pipettenspitze diese einen konischen Abschnitt aufweist, über welchen ein komplementärer konischer Abschnitt am oberen Teil der Klonierglocke gestülpt und der Werkzeugkopf an einem Arm eines Robotsystems befestigt ist,
**dadurch gekennzeichnet, dass**
die Vorrichtung am werkzeugkopfseitigen Ende des Armes einen Aufnahmeblock (1) aufweist, welcher einen unteren und einen oberen Anschlag (2, 3) als Verschiebewegbegrenzung einer beweglichen Halterung (4) zur Pipettenspitzenaufnahme aufweist, wobei die Halterung (4) am Aufnahmeblock (1) mittels einer Linearführung (5) geführt ist, der obere Anschlag (3) als gestaffelte, einseitig eingespannte Blattfederanordnung (12, 13, 14) ausgeführt ist, so dass sich über den konstant vorgegebenen Verschiebeweg bei einer Bewegung des Armes mit Werkzeugkopf gegen einen Fixpunkt verschiedene, gestufte Anschlagskräfte ergeben, welche einen definierten Kraftschluss der konischen Halterungen von Pipettenspitze und Klonierglocke ergeben.

2. Vorrichtung nach Anspruch 1,
**dadurch gekennzeichnet, dass**
die Pipettenspitzenhalterung (4) einen Hohlzylinder (6) mit Schlauchanschluss (7) umfasst, wobei der Hohlzylinder (6) am unteren Ende außenumfangsseitig konisch ausgebildet ist.

3. Vorrichtung nach Anspruch 1 oder 2,
**dadurch gekennzeichnet, dass**
im Aufnahmeblock (1) Bolzen (10) zur Befestigung der Blattfedern (12, 13, 14) vorgesehen sind.

4. Vorrichtung nach Anspruch 3,
**dadurch gekennzeichnet, dass**
die Bolzen (10) Hülsenteile (11, 11.1) aufnehmen, welche Abstandshalter für die Blattfedern darstellen.

5. Vorrichtung nach Anspruch 4,
**dadurch gekennzeichnet, dass**
eine erste, untere Blattfeder (12) unmittelbar am Aufnahmeblock (1) aufliegt und zur beweglichen Halterung (4) hin gerichtet eine Kröpfung aufweist.

6. Vorrichtung nach Anspruch 4,
**dadurch gekennzeichnet, dass**
eine zweite Blattfeder (13) über ein erstes Hülsenteil (11) gehalten und eine dritte Blattfeder (14) oberhalb der zweiten Blattfeder (13) über ein zweites Hülsenteil (11.1) abgestützt ist.

7. Vorrichtung nach einem der vorangegangenen Ansprüche,
**dadurch gekennzeichnet, dass**
die Blattfedern (12, 13, 14) jeweils als seitlich beabstandete Federpaare ausgebildet sind.

8. Vorrichtung nach Anspruch 5 und 6,
**dadurch gekennzeichnet, dass**
bei Biegebelastung der ersten, unteren Blattfeder (12) ein Kröpfungsabschnitt mit dem darüber liegenden freien Ende der zweiten Blattfeder (13) in Kontakt kommt und sich deren Biegebewegung auf die dritte, darüber liegende Blattfeder (14) überträgt.

## Claims

1. Device for automatically isolating and treating cell clones using a cloning dome for separating the respective cell clone from the environment, which device has a tool head, wherein the cloning dome is held releasably by a pipette tip and the latter is fixed by a force fit to a conical socket (8) of the tool head, the pipette tip furthermore has a conical section for holding the cloning dome on the pipette tip, over which section a complementary conical section on the upper part of the cloning dome is pulled, and the tool head is fastened to an arm of a robot system,
**characterised in that**
the device has a socket block (1) on the tool head-side end of the arm, which socket block has a lower and an upper stop (2, 3) as the displacement travel limiter of a movable holder (4) for receiving the pipette tip, wherein the holder (4) is guided on the socket block (1) by means of a linear guide (5), the upper stop (3) is configured as a stepped flat spring arrangement (12, 13, 14) which is clamped on one side, so that different, graduated stop forces are produced over the displacement travel, which is predefined to be constant, when the arm with the tool head moves towards a fixed point, which forces produce a defined force fit of the conical holders of the pipette tip and the cloning dome.

2. Device according to Claim 1,
**characterised in that**
the pipette tip holder (4) comprises a hollow cylinder (6) with a hose connection (7), wherein the hollow cylinder (6) has a conical configuration on the outer circumferential side at the lower end.

3. Device according to Claim 1 or 2,
**characterised in that**
bolts (10) are provided in the socket block (1) for fastening the flat springs (12, 13, 14).

4. Device according to Claim 3,
**characterised in that**
the bolts (10) accommodate sleeve parts (11, 11.1) which constitute spacers for the flat springs.

5. Device according to Claim 4, **characterised in that**
a first, lower flat spring (12) bears directly against the socket block (1) and has a projection directed towards the movable holder (4).

6. Device according to Claim 4,
**characterised in that**
a second flat spring (13) is held by means of a first sleeve part (11) and a third flat spring (14) is supported by means of a second sleeve part (11.1) above the second flat spring (13).

7. Device according to one of the preceding claims,
**characterised in that**
the flat springs (12, 13, 14) are in each case configured as laterally separated spring pairs.

8. Device according to Claims 5 and 6,
**characterised in that**
in the event of bending of the first, lower flat spring (12), a projection section comes into contact with the free end of the second flat spring (13), which end lies above it, and transmits its bending movement to the third flat spring (14) which lies above it.

## Revendications

1. Dispositif pour l'isolation et le traitement automatiques de clones cellulaires, au moyen d'une cloche de clonage pour la séparation de chaque clone cellulaire par rapport à l'environnement, comportant une tête d'outil, la cloche de clonage étant maintenue de façon amovible par une pointe de pipette, et cette dernière étant fixée par blocage à une admission conique (8) de la tête d'outil, celle-ci comportant une section conique pour le maintien de la cloche de clonage sur la pointe de pipette, par le biais de laquelle une section conique complémentaire est enfoncée sur la partie supérieure de la cloche de clonage et la tête d'outil est fixée sur un bras du système de robot,
**caractérisé en ce que**
le dispositif comporte un bloc d'admission (1) à l'extrémité du bras du côté de la tête d'outil, comportant une butée inférieure et une butée supérieure (2, 3) en tant que délimitations du déplacement d'un support mobile (4) par rapport à l'admission de pointe de pipette, le support (4) étant guidé sur le bloc d'admission (1) au moyen d'un guidage linéaire (5), la butée supérieure (3) étant conçue comme un ensemble de ressorts à lame (12, 13, 14) échelonnés, maintenu d'un côté par serrage, de sorte que différentes forces de butée échelonnées apparaissent lors d'un mouvement du bras avec la tête d'outil le long du trajet de déplacement défini constant, vers un point fixe, ces forces de butée produisant une liaison à force définie entre les supports coniques de la pointe de pipette et de la cloche de clonage.

2. Dispositif selon la revendication 1,
**caractérisé en ce que**
le support de pointe de pipette (4) comprend un cylindre creux (6) avec un raccord de tube (7), le cylindre creux (6) présentant une forme conique à l'extrémité inférieure du côté du pourtour extérieur.

3. Dispositif selon l'une des revendications 1 ou 2,
**caractérisé en ce que**
des boulons (10) destinés à la fixation des ressorts à lame (12, 13, 14) sont prévus dans le bloc d'admission (1).

4. Dispositif selon la revendication 3,
**caractérisé en ce que**
les boulons (10) accueillent des partie de douille (11, 11.1) constituant des écarteurs pour les ressorts à lame.

5. Dispositif selon la revendication 4,
**caractérisé en ce que**
un premier ressort à lame (12) inférieur est appliqué directement sur le bloc d'admission (1) et comporte un coude orienté vers le support mobile (4).

6. Dispositif selon la revendication 4,
**caractérisé en ce que**
un deuxième ressort à lame (13) est maintenu par une première partie de douille (11) et un troisième ressort à lame (14) situé au-dessus du deuxième ressort à lame (13) est s'appuie sur une deuxième partie de douille (11.1).

7. Dispositif selon l'une des revendications précédentes,
**caractérisé en ce que**
les ressorts à lame (12, 13, 14) sont conçus comme des paires de ressorts respectives, espacées latéralement.

8. Dispositif selon les revendications 5 et 6,
**caractérisé en ce que**
lors d'une charge par flexion du premier ressort à lame inférieur (12), une section de coude entre en contact avec l'extrémité libre du deuxième ressort à lame (13), située au-dessus, et son mouvement de flexion se transmet au troisième ressort à lame (14) situé au-dessus.
